# FASCICULE DE BREVET EUROPEEN

(11) **EP 0 435 749 B1**
(45) Date de publication et mention de la délivrance du brevet: **10.01.1996**
(21) Numéro de dépôt: 90403689.4
(22) Date de dépôt: 20.12.1990
(51) Int. Cl.: C07D 239/42, A61K 31/505

(54) **Dérivés de 2-aminopyrimidine-4-carboxamide, leur préparation et leur application en thérapeutique**
2-Aminopyrimidin-4-carboxamid-Derivate, deren Herstellung und deren therapeutische Anwendung
Derivatives of 2-aminopyrimidine-4-carboxamide, their preparation and therapeutic use

(30) Priorité: 28.12.1989 FR 8917304
(43) Date de publication de la demande: 03.07.1991
(73) Titulaire: SYNTHELABO, F-92350 Le Plessis Robinson (FR)
(72) Inventeur: George, Pascal, F-78730 St Arnoult en Yvelines (FR); Manoury, Philippe, F-91370 Verrières le Buisson (FR); Froissant, Jacques, F-41160 Morée (FR); Merly, Jean-Pierre, F-92330 Sceaux (FR)
(74) Mandataire: Ludwig, Jacques

(56) Documents cités:
- DE-A- 2 143 730

## Description

La présente invention a pour objet des dérivés de 2-aminopyrimidine-4-carboxamide, leur préparation et leur application en thérapeutique.

Les composés de l'invention répondent à la formule générale I du schéma donné ci-après, dans laquelle
n représente le nombre 2, 3, 4 ou 5,
p représente le nombre 0 ou 1,
R₁ représente un atome d'hydrogène ou un groupe méthyle,
Xₘ représente un ou plusieurs atomes ou groupes choisis parmi les suivants : hydrogène, fluor, chlore, méthoxy, isopropyle, cyclopropyle.

Ils peuvent se présenter à l'état de bases libres ou de sels d'addition à des acides.

Des composés de structure chimique proche de celle des composés de l'invention sont décrits dans la demande de brevet DE-2139083 ; toutefois ce document ne décrit qu'une activité antihypertensive des composés connus, et non pas une activité au niveau du bas appareil urinaire.

Conformément à l'invention on peut préparer les composés de formule générale (I) selon le procédé illustré par le schéma 1 ci-après.
On fait réagir une pipérazine éventuellement substituée de formule générale (II) (dans laquelle Xₘ et p sont tels que définis ci-dessus) avec un réactif halogéné de formule générale (III) (dans laquelle Y représente un atome d'halogène, n est tel que défini ci-dessus et, soit R₁ est tel que défini ci-dessus et R représente un groupe protecteur de l'amine, par exemple un groupe triphénylméthyle, soit R₁ et R forment ensemble un groupe protecteur tel que le groupe phthalimide comme décrit dans J. Med. Chem. (1988), 31(10), 1968-1971, J. Med. Chem., (1989), 32(8), 1921-1926, et Chem. Pharm. Bull (1989), 37(1), 100-105). La réaction s'effectue dans un solvant aprotique, tel que le diméthylformamide, en présence d'une base, organique telle que la triéthylamine ou minérale telle que le carbonate de potassium, à une température de 40 à 80°C.
On obtient une pipérazine de formule générale (IV), dont on réalise ensuite la déprotection de l'alkylamine terminale : dans le cas où R est un groupe triphénylméthyle on effectue un traitement à l'acide chlorhydrique gazeux dans un alcool aliphatique, par exemple le méthanol, à une température de 0 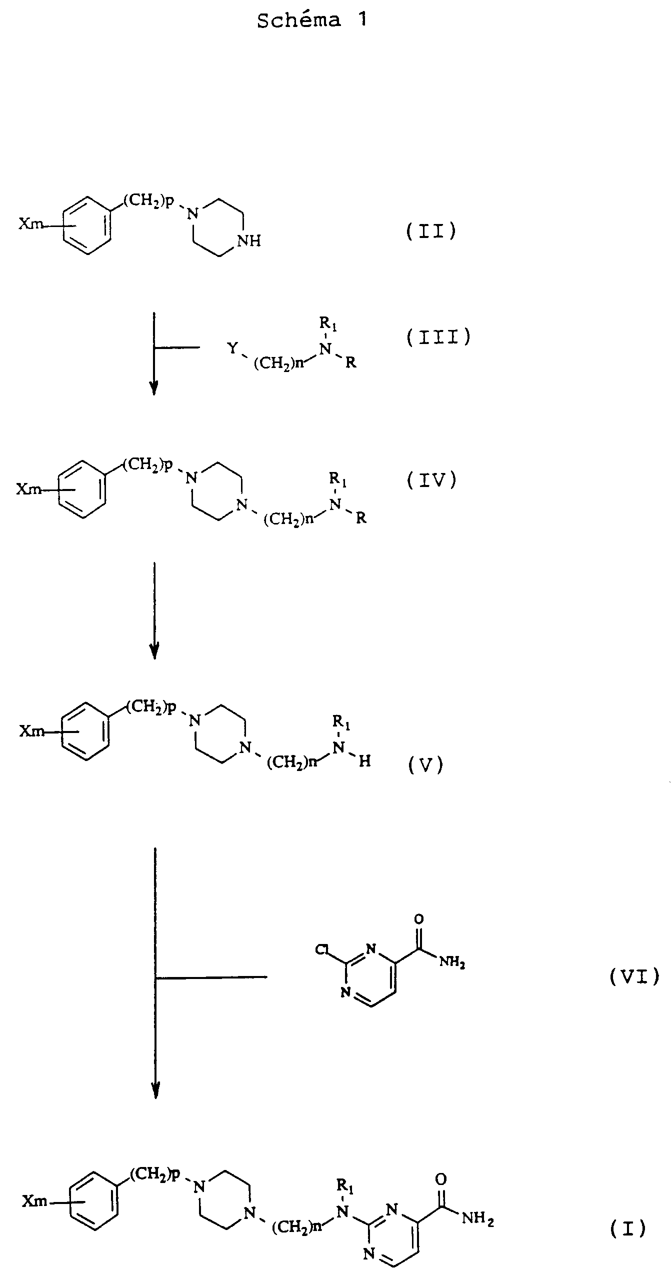
à 60°C; dans le cas où R et R₁ forment ensemble un groupe phtalimide on effectue un traitement analogue à celui décrit dans la littérature citée ci-dessus, par exemple avec de l'hydrazine.
On obtient ainsi l'amine de formule générale (V) que l'on fait réagir avec le 2-chloropyrimidine-4-carboxamide de formule (VI), dans un solvant aprotique, par exemple le diméthylformamide, en présence d'une base, par exemple le carbonate de potassium, à une température de 20 à 40°C, pour donner un dérivé de 2-aminopyrimidine-4-carboxamide de formule générale (I).

Certains composés de formule générale (V) dans laquelle p=0 sont décrits dans les brevets DE-2143730, DE-2314114, DE-3524635, US-3398151 et US-4748240.

Le réactif halogéné de formule générale (III) est soit disponible dans le commerce quand R₁ et R forment ensemble un groupe phtalimido, soit, quand R₁ est H ou CH₃, peut être préparé suivant le schéma 2, donné ci-après, et selon lequel on fait réagir une ω-halogénoalkylamine de formule générale (VII) avec un composé de formule RCl, en l'occurence le chlorure de trityle, dans un solvant inerte halogéné tel que le dichlorométhane, en présence d'une base organique telle que la triéthylamine, à une température de 20 à 80°C.

Le 2-chloropyrimidine-4-carboxamide de formule (VI) peut être préparé selon le schéma 3, donné ci-après, à partir du 2-chloropyrimidine-4-carbonitrile de formule (VIII) par traitement à l'acide chlorhydrique gazeux dans l'acide formique. Le 2-chloropyrimidine-4-carbonitrile est préparé selon la méthode décrite dans J. Het. Chem. (1964), 1, 130-133.

Les exemples suivants illustrent en détail la préparation de quelques composés selon l'invention. Les numéros indiqués entre parenthèses dans les titres des exemples correspondent à ceux du tableau donné plus loin.
Les microanalyses élémentaires et les spectres IR et RMN confirment les structures des produits obtenus.

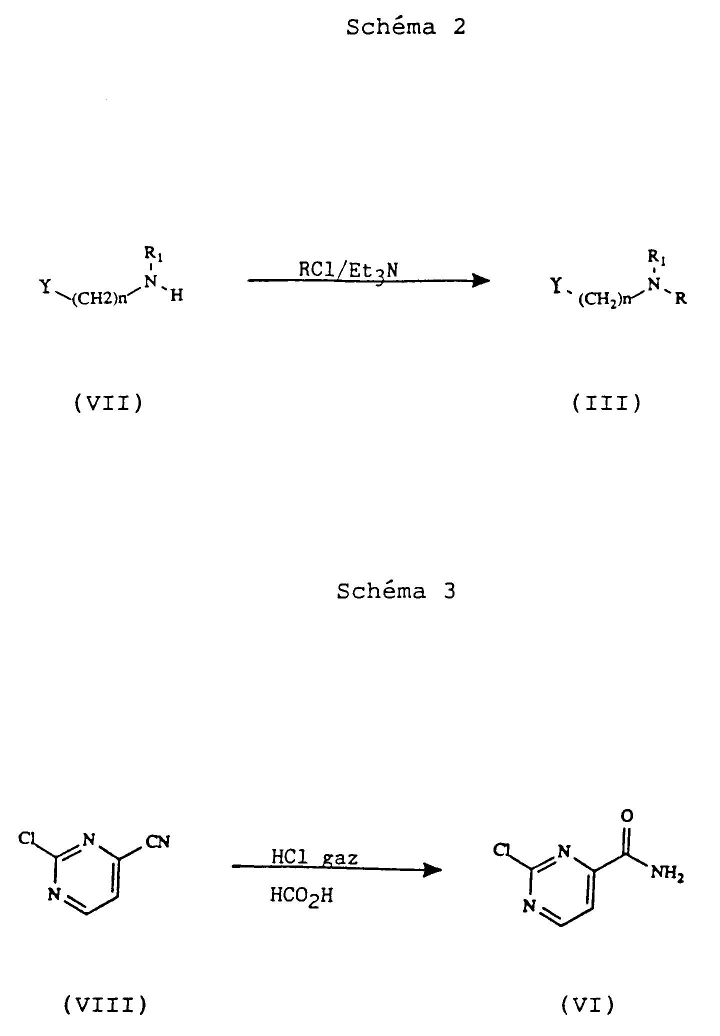

### Exemple 1 (Composé N°5)

### 2-[[2-[4-(2-Méthoxyphényl)pipérazin-1-yl]éthyl]amino]pyrimidine-4-carboxamide, fumarate.

### a) 2-Bromo-N-(triphénylméthyl)éthanamine.

Dans un ballon de 2 l placé sous azote on introduit 272,1 g (976 mmoles) de triphénylchlorométhane, 800 ml de dichlorométhane et 200 g (976 mmoles) de bromhydrate de 2-bromoéthanamine. On agite le mélange et on ajoute, goutte à goutte, 300 ml de triéthylamine, et on maintient l'agitation pendant 48h. On sépare le chlorhydrate de triéthylamine qui précipite, on ajoute de l'eau au filtrat, on sépare la phase organique, on la lave à l'eau, on la sèche sur sulfate de sodium, et on évapore le solvant. On obtient une huile qu'on purifie par chromatographie sur colonne de gel de silice. Après recristallisation dans un mélange de dichlorométhane et de cyclohexane on obtient 273,17 g de solide blanc. Point de fusion: 108-109°C.

### b) 2-[4-(2-Méthoxyphényl)pipérazin-1-yl]-N-(triphénylméthyl)-éthanamine.

Dans un ballon de 500 ml muni d'un réfrigérant et placé sous azote on introduit 10 g (27,3 mmoles) de 2-bromo-N-(triphénylméthyl)éthanamine, 200 ml d'acétonitrile, 5,15 g (27,3 mmoles) de 1-(2-méthoxyphényl)pipérazine, 5,6 g de carbonate de potassium anhydre, quelques grains d'iodure de sodium, et 1 ml de diméthylformamide. On chauffe le mélange au reflux pendant 15h, on évapore les solvants, on ajoute de l'eau et du dichlorométhane, on sépare la phase organique, on la lave à l'eau, on la sèche sur sulfate de sodium et on chasse le solvant. On obtient une huile visqueuse qu'on purifie par chromatographie sur colonne de gel de silice en éluant avec un mélange d'acétate d'éthyle et de dichlorométhane. On isole 9,24 g de produit.

### c) 2-[4-(2-Méthoxyphényl)pipérazin-1-yl]éthanamine, trichlorhydrate.

On ajoute 400 ml de méthanol au composé précédent et, après homogénéisation, on fait barboter dans la solution de l'acide chlorhydrique gazeux pendant 10mn. On filtre le précipité formé, on le rince au méthanol et on le sèche sous vide. On obtient 5,33 g de solide blanc.

### d) 2-[[2-[4-(2-Méthoxyphényl)pipérazin-1-yl]éthyl]amino]pyrimidine-4-carboxamide, fumarate.

Dans un ballon de 500 ml muni d'un réfrigérant et placé sous azote on introduit 5,33 g (15,36 mmoles) de trichlorhydrate de 2-[4-(2-méthoxyphényl)pipérazin-1-yl]éthanamine, 2,44 g (15,36 mmoles) de 2-chloropyrimidine-4-carboxamide, 200 ml d'acétonitrile et 12 ml de triéthylamine. On chauffe le mélange au reflux pendant 12h, on le laisse refroidir, on évapore le solvant, on ajoute au résidu de l'eau et du dichlorométhane, on sépare la phase organique, on la lave à l'eau, on la sèche sur sulfate de sodium, on évapore le solvant, on reprend le résidu avec de l'acétate d'éthyle, on sépare une impureté par filtration, et on évapore le filtrat. On obtient 1,86 g de solide.
Point de fusion : 185-187°C (décomposition).
On en introduit 1,85 g (5,19 mmoles) dans un ballon de 1 l, on ajoute 0,602 g (5,19 mmoles) d'acide fumarique, et on dissout le tout avec du méthanol chaud. On filtre la solution, on la concentre et on la laisse refroidir. On filtre le précipité, on le recristallise dans le méthanol et on le sèche sous vide. On isole finalement 1,58 g de fumarate.
Point de fusion : 224-225°C.

### Exemple 2 (Composé N°11).

### 2-[[2-[4-(2-Cyclopropylphényl)pipérazin-1-yl]éthyl]méthylamino]pyrimidine-4-carboxamide, fumarate.

### a) 2-Chloro-N-méthyl-N-(triphénylméthyl)éthanamine.

Dnas un ballon de 2 l on introduit 138,95 g (1,85 mole) de 2-(méthylamino)éthanol et 500 ml de dichlorométhane et, pendant deux fois 5mnn, à 30mn d'intervalle, on fait barboter de l'acide chlorhydrique gazeux. On concentre partiellement la solution puis on ajoute lentement, en 1h30, 145 ml (1,98 mole) de chlorure de thionyle. On agite le mélange pendant 10h à température ambiante puis on le chauffe à 50°C pendant 2h. On laisse refroidir, on évapore le solvant et on reprend le résidu deux fois avec du toluène, qu'on évapore. On rince le résidu avec un mélange d'éther diéthylique et de pentane et on le sèche sous vide en présence d'anhydride phosphorique. On obtient 230,18 g de chlorhydrate de 2-chloro-N-méthyléthanamine. On en place 46,17 g (355,1 mmoles) dans un ballon de 1 l placé sous azote et muni d'une ampoule à brome, on ajoute 100 g (358,7 mmoles) de triphénylchlorométhane et 400 ml de dichlorométhane, et on introduit lentement 100 ml de triéthylamine. On maintient l'agitation pendant 2 jours, puis on ajoute de l'eau, on sépare la phase organique, on la lave à l'eau, on la sèche sur sulfate de sodium et on évapore le solvant. On purifie le résidu par chromatographie sur colonne de gel de silice en éluant avec un mélange de dichlorométhane et de cyclohexane et, après recristallisation dans du cyclohexane on obtient 79 g de composé.
Point de fusion : 161-163°C.

### b) 2-[4-(2-Cyclopropylphényl)pipérazin-1-yl]-N-méthyléthanamine, trichlorhydrate.

Dans un ballon de 500 ml muni d'un réfrigérant et placé sous azote on introduit 9,0 g (44,4 mmoles) de 1-(2-cyclopropylphényl)pipérazine, 200 ml de diméthylformamide, 15 g (44,4 mmoles) de 2-chloro-N-méthyl-N-(triphénylméthyl)éthanamine et 9 g de carbonate de potassium, et on chauffe le mélange trois fois pendant 6h à 96°C. On évapore le solvant, on reprend le résidu avec de l'eau et du dichlorométhane, on sépare la phase organique, on la lave à l'eau, on la sèche sur sulfate de sodium et on chasse le solvant. On obtient 4,17 g de 2-[4-(2-cyclopropylphényl)pipérazin-1-yl]-N-méthyl-N-(triphénylméthyl)éthanamine sous forme d'huile qu'on dissout dans 200 ml de méthanol, on y fait barboter de l'acide chlorhydrique gazeux pendant 10mn, on concentre le mélange, on le laisse reposer 2 jours et on sépare le précipité par filtration. On obtient 2,94 g de composé.

### c) 2-[[2-[4-(2-Cyclopropylphényl)pipérazin-1-yl]éthyl]méthylamino]pyrimidine-4-carboxamide, fumarate.

Dans un ballon de 500 ml muni d'un réfrigérant et placé sous azote on introduit 2,7 g (7,31 mmoles) de trichlorhydrate de 2-[4-(2-cyclopropylphényl)pipérazin-1-yl]-N-méthyléthanamine, 1,15 g (7,31 mmoles) de 2-chloropyrimidine-4-carboxamide, 8 g de carbonate de potassium et 200 ml d'acétonitrile, et on chauffe le mélange au reflux pendant 12h. On le laisse refroidir, on évapore le solvant, on reprend le résidu avec de l'eau et du dichlorométhane, on sépare la phase organique, on la lave à l'eau, on la sèche sur sulfate de sodium, on évapore le solvant et on purifie le résidu par chromatographie sur colonne de gel de silice. Après recristallisation on obtient 2,223 g de composé sous forme de base libre.
Point de fusion : 162,5-163,5°C.
On en prépare le fumarate en ajoutant 0,678 g (5,84 mmoles) d'acide fumarique puis du méthanol à 80°C jusqu'à dissolution, on filtre le mélange, on le concentre et on le laisse refroidir. On isole le précipité par filtration, on le rince au méthanol puis à l'éther diéthylique, et on le recristallise dans le méthanol. On isole finalement 1,67 g de fumarate.
Point de fusion : 177-180°C.

### Exemple 3 (Composé N°14)

### 2-[[3-[4-(3-Chlorophényl)pipérazin-1-yl]propyl]amino]pyrimidine-4-carboxamide, fumarate.

### a) 2-chloropyrimidine-4-carboxamide.

Dans un ballon de 1 l muni d'une agitation magnétique et contenant 131,9 g (946 mmoles) de 2-chloropyrimidine-4-carbonitrile, on introduit 338 g d'acide formique à 98%, et on fait passer lentement un courant d'acide chlorhydrique gazeux pendant 1h30. On laisse reposer le mélange pendant la nuit, on isole le solide sur verre fritté, puis on le purifie par recristallisation avec filtration à chaud dans un mélange de nitrométhane et d'acétate d'éthyle. On isole ainsi, en trois jets, et après séchage sous vide et à chaud, 107,03 g de solide gris. Point de fusion : 152,5-154°C.

### b) 3-Bromo-N-(triphénylméthyl)propanamine.

A 153,3 g (0,7 mole) de bromhydrate de 3-bromopropylamine en solution dans 200 ml de dichlorométhane on ajoute 139,5 g (0,5 mole) de chlorotriphénylméthane en solution dans 200 ml de dichlorométhane. On ajoute alors lentement, en l'espace de 3h, 132,0 g, soit 180,8 ml (1,3 mole) de triéthylamine. On obtient un mélange hétérogène blanchâtre qu'on agite pendant 14 h.
On la verse dans de l'eau, on sépare la phase organique, on la lave à l'eau, on la sèche et on évapore le solvant, ce qui donne 220,5 g de produit huileux. On reprend le résidu avec un mélange 1/1 de cyclohexane/toluène, et on sépare un insoluble par décantation. On réduit la phase organique de moitié en volume, et on ajoute lentement de l'éther de pétrole. Par trituration on obtient 111,2 g de solide blanc.
Point de fusion : 100-102,5°C.

### c) 3-[4-(3-chlorophényl)pipérazin-1-yl]-N-(triphénylméthyl)propanamine.

On agite à 90°C, pendant 6,5 h et sous argon, un mélange de 5,9 g (30 mmoles) de 1-(3-chlorophényl)pipérazine, 12,55 g (33 mmoles) de 3-bromo-N-(triphénylméthyl)propanamine, 6,2 g (45 mmoles) de carbonate de potassium et 60 ml de diméthylformamide. Puis on verse le mélange dans de l'eau glacée, et on l'extrait à l'acétate d'éthyle. On sépare la phase organique, on la lave à l'eau, on la sèche et on évapore le solvant. On obtient 15,7 g de résidu huileux qu'on purifie par chromatographie sur colonne de gel de silice en éluant avec un mélange 99/1 de dichlorométhane/méthanol, ce qui donne 11,8 g d'huile jaune clair.

### d) 3-[4-(3-chlorophényl)pipérazin-1-yl]propanamine.

Dans un ballon contenant 11,2 g (22,6 mmoles) de 3-[4-(3-chlorophényl)pipérazin-1-yl]-N-(triphénylméthyl)propanamine et 300 ml de méthanol on fait passer de l'acide chlorhydrique gazeux pendant 10 mn, tout en refroidissant le mélange par un bain d'eau glacée. On laisse le mélange revenir à température ambiante, puis on le chauffe au reflux pendant 5,5 h. On réduit la solution à la moitié de son volume et on laisse refroidir. On filtre le solide qui précipite, on le traite avec de l'eau et de l'ammoniaque, on extrait le mélange à l'acétate d'éthyle, on lave la phase organique à l'eau, on la sèche et on évapore le solvant. On obtient 3,9 g d'huile jaune clair.

### e) 2-[[3-[4-(3-Chlorophényl)pipérazin-1-yl]propyl]amino]pyrimidine-4-carboxamide, fumarate.

Dans un ballon de 100 ml, sous atmosphère d'argon, on introduit 3,9 g (15,4 mmoles) de 3-[4-(3-chlorophényl)-pipérazin-1-yl]propanamine, 2,42 g (15,4 mmoles) de 2-chloropyrimidine-4-carboxamide, 70 ml de diméthylformamide et une quantité catalytique d'iodure de sodium. On ajoute encore 2,34 g (16,9 mmoles) de carbonate de potassium, et on agite le mélange à température ambiante pendant 19 h. Puis on traite le mélange avec de l'eau, on l'extrait à l'acétate d'éthyle, on lave l'extrait organique à l'eau, on le sèche et on évapore le solvant. On obtient une huile orange qu'on triture dans l'acétate d'éthyle. Après filtration et séchage, on isole 2,2 g de solide blanc.
On en prépare le fumarate en le solubilisant dans 100 ml d'éthanol et en y ajoutant 0,68 g d'acide fumarique en solution dans 100 ml d'éthanol, puis 100 ml d'éthanol. On chauffe le mélange au reflux, on évapore l'éthanol, on triture l'huile résiduelle dans l'acétate d'éthyle, et on recristallise le solide obtenu dans l'éthanol. On isole finalement 2,3 g de fumarate.
Point de fusion : 187-189°C.

### Exemple 4 (Composé N°12).

### 2-[[3-[4-(2-Cyclopropylphényl)pipérazin-1-yl]propyl]méthylamino]pyrimidine-4-carboxamide.

### a) 3-[4-(2-Cyclopropylphényl)pipérazin-1-yl]-N-méthylpropanamine.

On opère de manière analogue à celle décrite dans l'exemple 2 (a et b), en partant de 3-(méthylamino)propanol.

### b) 2-[[3-[4-(2-Cyclopropylphényl)pipérazin-1-yl]propyl]méthylamino]pyrimidine-4-carboxamide.

Dans un ballon de 250 ml muni d'un réfrigérant et d'une garde à chlorure de calcium on introduit 1,25 g (4,09 mmoles) de 3-[4-(2-cyclopropylphényl)pipérazin-1-yl]-N-méthylpropanamine, 150 ml d'acétonitrile, 0,645 g (4,09 mmoles) de 2-chloropyrimidine-4-carboxamide et 1 g de carbonate de potassium. On chauffe le mélange au reflux pendant 6h, on le laisse revenir à température ambiante, on le concentre partiellement, et on ajoute de l'eau et du dichlorométhane. On sépare la phase organique, on la lave à l'eau, on la sèche sur sulfate de sodium, on évapore le solvant et on purifie le résidu par chromatographie sur colonne de gel de silice. Le produit huileux obtenu cristallise dans l'acétonitrile, on le sépare par filtration, on le rince à l'éther diéthylique et on le sèche à chaud sous vide. On isole finalement 0,97 g de composé.
Point de fusion : 169,5-170,5°C.

Le tableau ci-après illustre les structures et propriétés physiques de quelques composés selon l'invention.

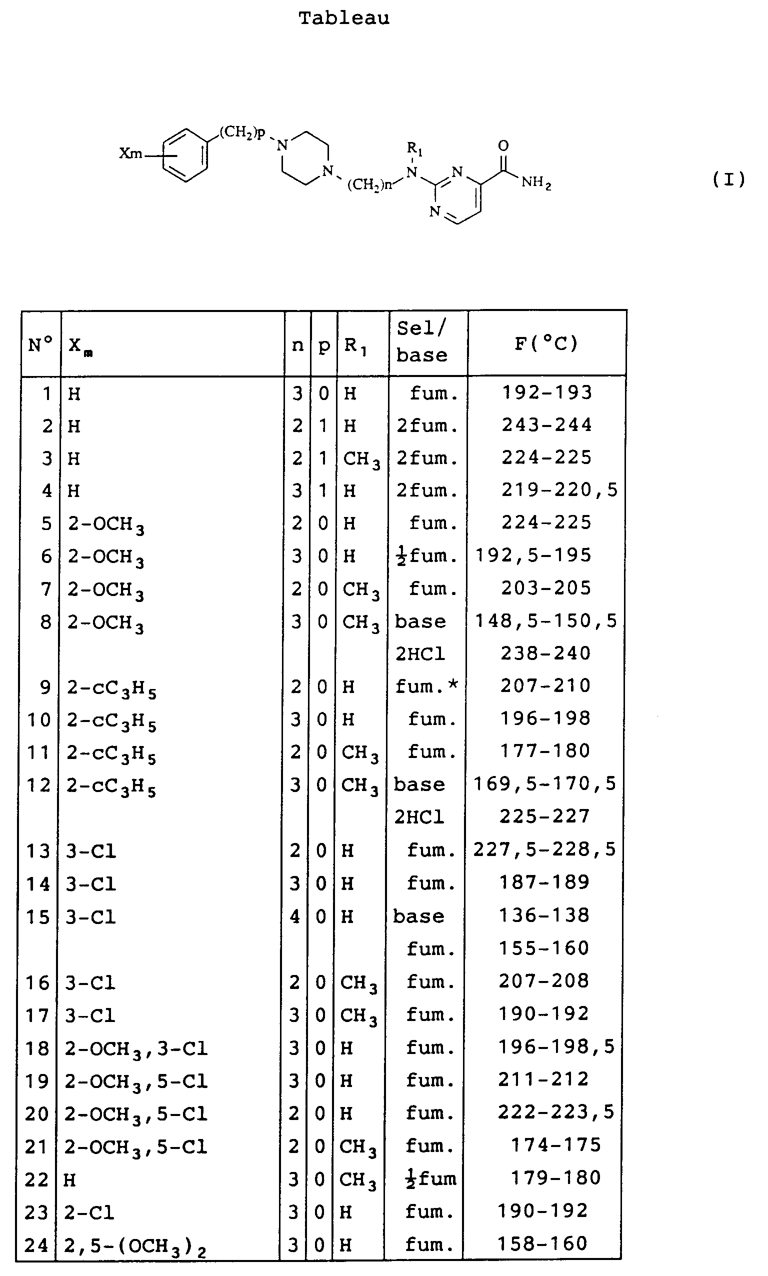

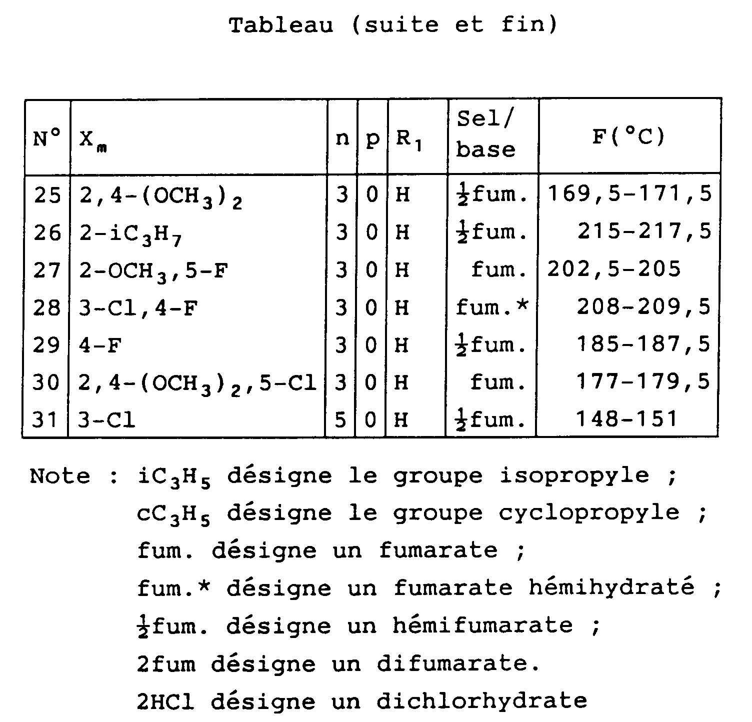

Les composés de l'invention ont été soumis à une série d'essais pharmacologiques qui ont mis en évidence leur intérêt comme substances à activités thérapeutiques.

L'activité antihypertensive des composés de l'invention a été étudiée chez le rat spontanément hypertendu.
On place les rats, mâles et agés de 10 mois environ, pendant 20 mn dans une enceinte climatisée à 28°C, à 60% d'humidité relative, et on mesure leur pression artérielle systolique et leur fréquence cardiaque par captage piézoélectrique du pouls au niveau de l'artère caudale. On contrôle la stabilité de la pression artérielle plusieurs fois avant d'administrer les composés, les moyennes des quatre dernières mesures de pression et de fréquence étant alors prises comme valeurs de référence.
Les animaux reçoivent, par voie sous-cutanée, les solutions de composés à étudier à des doses de 0,03 à 30 mg/kg, ou seulement le solvant, et on mesure les pressions artérielles et les fréquences cardiaques à 30 mn, 1h, 3h et 5h après l'injection.
Les doses minimales diminuant la pression artérielle se situent entre 0,1 et 30 mg/kg.

Les composés de l'invention ont également fait l'objet d'études quant à leur activité antagoniste des récepteurs adrénergiques du type α₁ au niveau du bas appareil urinaire.
Leur activité *in vitro* a été étudiée sur l'urètre isolé de lapin.
On prépare des anneaux d'urètre de lapin adulte selon la méthode de Ueda et al., Eur. J. Pharmacol., (1984), 103, 249-254, puis, après sensibilisation à la noradrénaline, on détermine la courbe concentration-réponse à la phényléphrine, en absence et en présence de composé à étudier.
On évalue la puissance de l'antagonisme α₁-adrénergique de chaque composé par calcul du pA₂, antilogarithme de la concentration molaire d'antagoniste en présence de laquelle la concentration d'agoniste doit être doublée pour engendrer le même effet qu'en son absence.
Les pA₂ des composés sont de l'ordre de 5,5 à 9.

L'activité *in vivo* des composés de l'invention a été étudiée quant à leur effet sur l'hypertonie urétrale engendrée par la stimulation du nerf hypogastrique chez le chat anesthésié. On anesthésie des chats mâles adultes au pentobarbital sodique, et on les prépare selon la méthode de Theobald, J. Auton. Pharmac., (1983), 3, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme α₁-adrénergique de chaque composé par calcul de la DI₅₀, dose qui inhibe de 50% l'hypertonie urétrale.
Les DI₅₀ des composés de l'invention sont de l'ordre de 0,01 à 3 mg/kg.

Les résultats des essais montrent que certains composés de l'invention possèdent une activité antihypertensive. Par ailleurs ils montrent, *in vitro*, une activité antagoniste des récepteurs α₁ adrénergique des muscles lisses du bas appareil urinaire (urètre) stimulés par un agoniste α₁-adrénergique (phényléphrine). *In vivo*, ils inhibent l'hypertonie urétrale engendrée par la stimulation nerveuse sympathique.

Les composés de l'invention peuvent donc être utilisés pour le traitement d'affections cardiovasculaires telles que l'hypertension artérielle. Ils peuvent aussi être utilisés pour le traitement symptomatique des maladies et affections impliquant une hyperactivité du système α-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 100 mg de substance active.

## Revendications (Revendications pour l'(les) Etat(s) contractant(s) suivant(s): AT, BE, CH, DE, DK, FR, GB, IT, LI, LU, NL, SE)

1. Composés de formule générale (I) 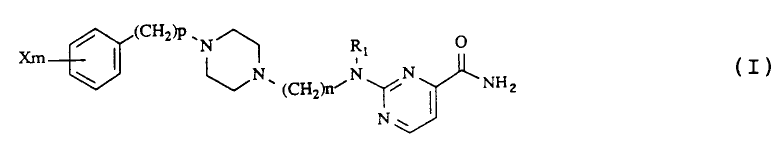 dans laquelle
n représente le nombre 2, 3, 4 ou 5,
p représente le nombre 0 ou 1,
R₁ représente un atome d'hydrogène ou un groupe méthyle,
Xₘ représente un ou plusieurs atomes ou groupes choisis parmi les suivants : hydrogène, fluor, chlore, méthoxy, isopropyle, cyclopropyle,
à l'état de bases libres ou de sels d'addition à des acides.

2. Procédé de préparation des composés selon la revendication 1, caractérisé en ce qu'on fait réagir un composé de formule générale (II) 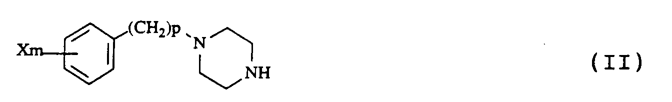 (dans laquelle Xₘ et p sont tels que définis dans la revendication 1)
avec un réactif halogéné de formule générale (III) 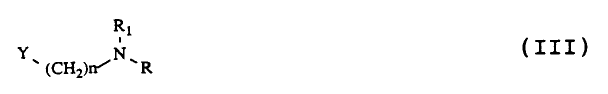 (dans laquelle Y représente un atome d'halogène, n est tel que défini dans la revendication 1 et, soit R₁ est tel que défini dans la revendication 1 et R représente un groupe protecteur de l'amine, par exemple un groupe triphénylméthyle, soit R₁ et R forment ensemble un groupe protecteur tel que le groupe phthalimide), dans un solvant aprotique, en présence d'une base organique ou minérale, à une température de 40 à 80°C, obtenant ainsi un composé de formule générale (IV) 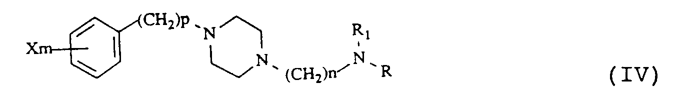 dont on réalise ensuite la déprotection de l'alkylamine terminale : dans le cas où R est un groupe triphénylméthyle on effectue un traitement à l'acide chlorhydrique gazeux dans un alcool aliphatique, à une température de 0 à 40°C ; et dans le cas où R et R₁ forment ensemble un groupe phtalimide on effectue un traitement avec de l'hydrazine, obtenant ainsi le composé de formule générale (V) 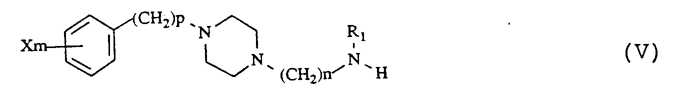 que l'on fait réagir avec le composé de formule (VI) 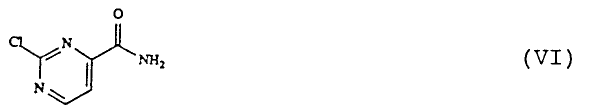 dans un solvant aprotique, en présence d'une base, à une température de 20 à 80°C.

3. Médicament, caractérisé en ce qu'il contient un composé selon la revendication 1.

4. Composé selon la revendication 1, en l'espèce le 2-[[3-[4-(5-chloro-2-méthoxyphényl)pipérazin-1-yl]propyl]amino]pyrimidine-4-carboxamide.

## Revendications (Revendications pour l'(les) Etat(s) contractant(s) suivant(s): ES, GR)

1. Procédé de préparation de composés de formule générale (I) 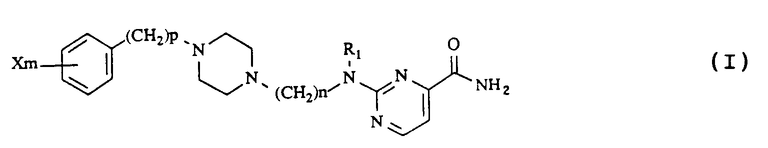 dans laquelle
n représente le nombre 2, 3, 4 ou 5,
p représente le nombre 0 ou 1,
R₁ représente un atome d'hydrogène ou un groupe méthyle,
Xₘ représente un ou plusieurs atomes ou groupes choisis parmi les suivants : hydrogène, fluor, chlore, méthoxy, isopropyle, cyclopropyle,
procédé caractérisé en ce qu'on fait réagir un composé de formule générale (II) 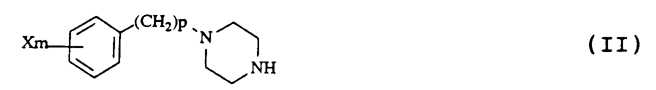 (dans laquelle Xₘ et p sont tels que définis ci-dessus) avec un réactif halogéné de formule générale (III) 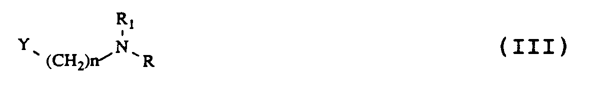 (dans laquelle Y représente un atome d'halogène, n est tel que défini ci-dessus et, soit R₁ est tel que défini ci-dessus et R représente un groupe protecteur de l'amine, par exemple un groupe triphénylméthyle, soit R₁ et R forment ensemble un groupe protecteur tel que le groupe phthalimide), dans un solvant aprotique, en présence d'une base organique ou minérale, à une température de 40 à 80°C, obtenant ainsi un composé de formule générale (IV) 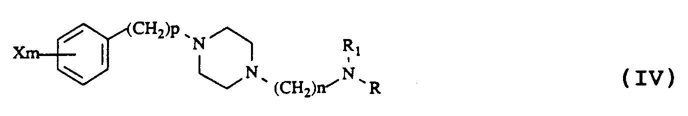 dont on réalise ensuite la déprotection de l'alkylamine terminale : dans le cas où R est un groupe triphénylméthyle on effectue un traitement à l'acide chlorhydrique gazeux dans un alcool aliphatique, à une température de 0 à 40°C ; et dans le cas où R et R₁ forment ensemble un groupe phtalimide on effectue un traitement avec de l'hydrazine, obtenant ainsi le composé de formule générale (V) 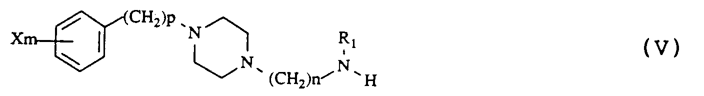 que l'on fait réagir avec le composé de formule (VI) 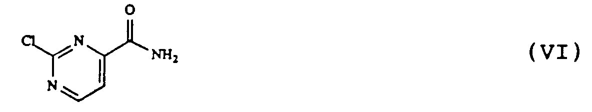 dans un solvant aprotique, en présence d'une base, à une température de 20 à 80°C.

## Claims (Claims for the following Contracting State(s): AT, BE, CH, DE, DK, FR, GB, IT, LI, LU, NL, SE)

1. Compounds of general formula (I) 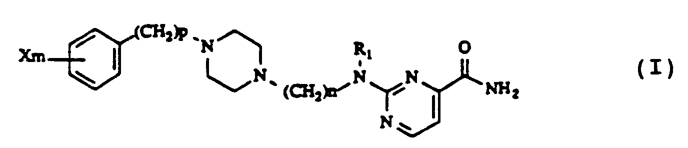 in which
n denotes the number 2, 3, 4 or 5,
p denotes the number 0 or 1,
R₁ denotes a hydrogen atom or a methyl group,
Xₘ denotes one or more atoms or groups chosen from the following: hydrogen, fluorine, chlorine, methoxy, isopropyl, cyclopropyl,
in the form of free bases or of acid addition salts.

2. Process for the preparation of the compounds according to Claim 1, characterised in that a compound of general formula (II) 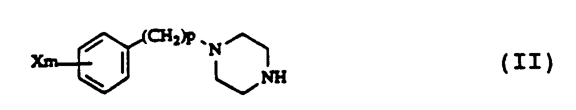 (in which Xₘ and p are such as defined in Claim 1) is reacted with a halogenated reactant of general formula (III) 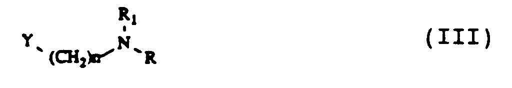 (in which Y denotes a halogen atom, n is such as defined in Claim 1 and either R₁ is such as defined in Claim 1 and R denotes an amine-protecting group, for example a triphenylmethyl group, or R₁ and R together form a protecting group such as the phthalimide group), in an aprotic solvent, in the presence of an organic or inorganic base, at a temperature of 40 to 80°C, thus obtaining a compound of general formula (IV) 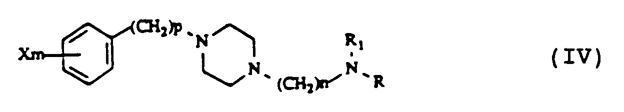 whose terminal alkylamine is subsequently deprotected: in the case where R is a triphenylmethyl group, a treatment with gaseous hydrochloric acid in an aliphatic alcohol is performed at a temperature of 0 to 40°C; and, in the case where R and R₁ together form a phthalimide group, a treatment with hydrazine is performed, thus obtaining the compound of general formula (V) 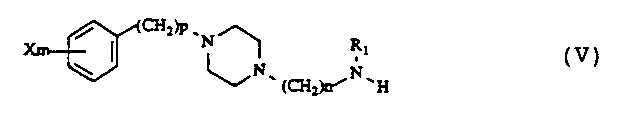 which is reacted with the compound of formula (VI) 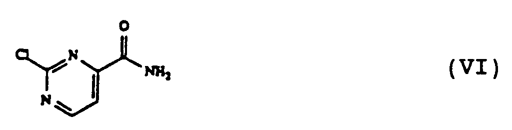 in an aprotic solvent, in the presence of a base, at a temperature of 20 to 80°C.

3. Medication characterized in that it contains a compound according to Claim 1.

4. Compound according to Claim 1, in this case 2-[[3-[4-(5-chloro-2-methoxyphenyl)piperazin-1-yl]propyl]amino]pyrimidine-4-carboxamide.

## Claims (Claims for the following Contracting State(s): ES, GR)

1. Process for the preparation of compounds of general formula (I) 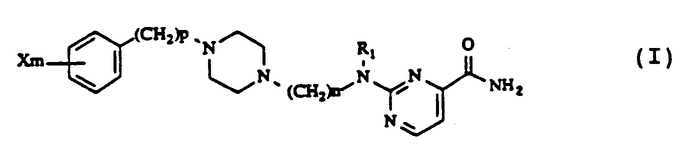 in which
n denotes the number 2, 3, 4 or 5,
p denotes the number 0 or 1,
R₁ denotes a hydrogen atom or a methyl group,
Xₘ denotes one or more atoms or groups chosen from the following: hydrogen, fluorine, chlorine, methoxy, isopropyl, cyclopropyl,
which process is characterised in that a compound of general formula (II) 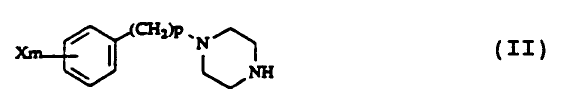 (in which Xₘ and p are such as defined above) is reacted with a halogenated reactant of general formula (III) 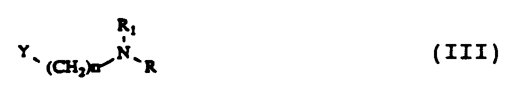 (in which Y denotes a halogen atom, n is such as defined above and either R₁ is such as defined above and R denotes an amine-protecting group, for example a triphenylmethyl group, or R₁ and R together form a protecting group such as the phthalimide group), in an aprotic solvent, in the presence of an organic or inorganic base, at a temperature of 40 to 80°C, thus obtaining a compound of general formula (IV) 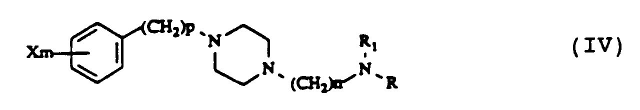 whose terminal alkylamine is subsequently deprotected: in the case where R is a triphenylmethyl group, a treatment with gaseous hydrochloric acid in an aliphatic alcohol is performed at a temperature of 0 to 40°C; and, in the case where R and R₁ together form a phthalimide group, a treatment with hydrazine is performed, thus obtaining the compound of general formula (V) 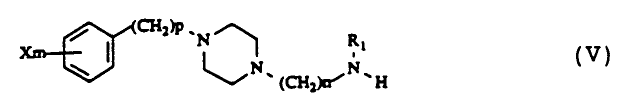 which is reacted with the compound of formula (VI) 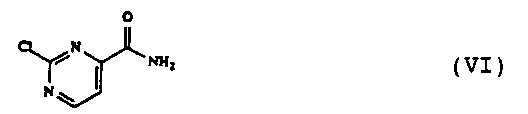 in an aprotic solvent, in the presence of a base, at a temperature of 20 to 80°C.

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel (I) 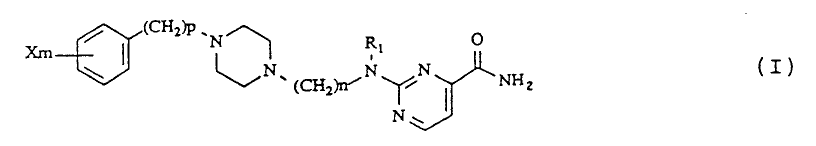 in der
n die Zahl 2, 3, 4 oder 5,
p die Zahl 0 oder 1,
R₁ ein Wasserstoffatom oder eine Methylgruppe und
Xₘ ein oder mehrere Atome oder Gruppen ausgewählt aus: Wasserstoff, Fluor, Chlor, Methoxy, Isopropyl und Cyclopropyl.
bedeuten,
in Form der freien Basen oder der Additionssalze mit Säuren.

2. Verfahren zur Herstellung der Verbindungen nach Anspruch 1, **dadurch gekennzeichnet**, daß man eine Verbindung der allgemeinen Formel (II) 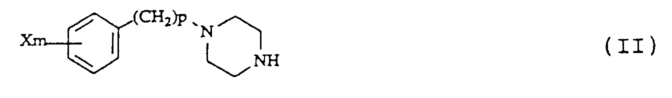 (in der Xₘ und p die in Anspruch 1 angegebenen Bedeutungen besitzen) mit einem Halogenreagens der allgemeinen Formel (III) 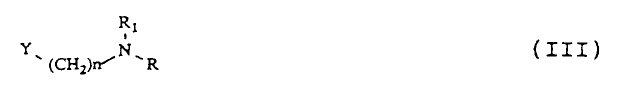 (in der Y ein Halogenatom darstellt, n die in Anspruch 1 angegebenen Bedeutungen besitzt und entweder R₁ die in Anspruch 1 angegebenen Bedeutungen besitzt und Reine Schutzgruppe für die Aminogruppe, beispielsweise eine Triphenylmethylgruppe, darstellt, oder R₁ und R gemeinsam eine Schutzgruppe, wie die Phthalimidgruppe, bedeuten) in einem aprotischen Lösungsmittel in Gegenwart einer organischen oder anorganischen Base bei einer Temperatur von 40 bis 80°C umsetzt zur Bildung einer Verbindung der allgemeinen Formel (IV) 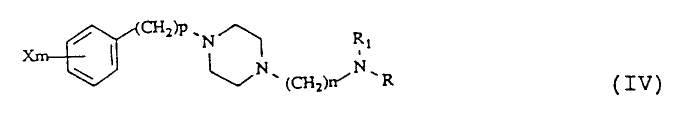 von der man anschließend die Schutzgruppe der endständigen Alkylamingruppe abspaltet: wobei dann, wenn R eine Triphenylmethylgruppe darstellt, man eine Behandlung mit gasförmiger Chlorwasserstoffsäure in einem aliphatischen Alkohol bei einer Temperatur von 0 bis 40°C durchführt; und dann, wenn R und R₁ gemeinsam eine Phthalimidgruppe bilden, man eine Behandlung mit Hydrazin durchführt, so daß man die Verbindung der allgemeinen Formel (V) 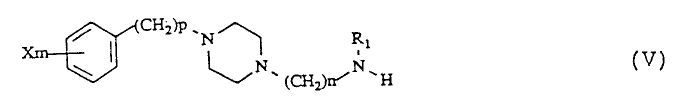 erhält, die man mit der Verbindung der Formel (VI) 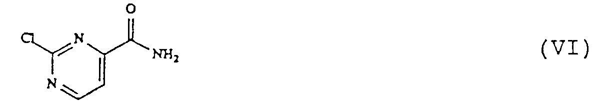 in Gegenwart einer Base bei einer Temperatur von 20 bis 80°C in einem aprotischen Lösungsmittel umsetzt.

3. Arzneimittel, **dadurch gekennzeichnet**, daß es eine Verbindung nach Anspruch 1 enthält.

4. Verbindung nach Anspruch 1, nämlich 2-[[3-[4-(5-Chlor-2-methoxyphenyl)-piperazin-1-yl]-propyl]-amino]-pyrimidin-4-carboxamid.

## Patentansprüche (Patentansprüche für folgende(n) Vertragsstaat(en): ES, GR)

1. Verfahren zur Herstellung der Verbindungen der allgemeinen Formel (I) 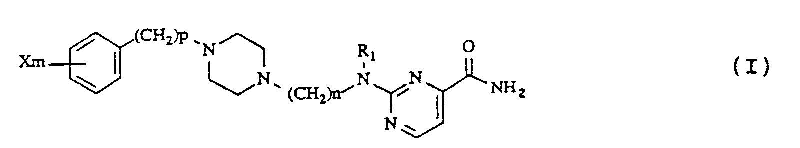 in der
n die Zahl 2, 3, 4 oder 5,
p die Zahl 0 oder 1,
R₁ ein Wasserstoffatom oder eine Methylgruppe und
Xₘ ein oder mehrere Atome oder Gruppen ausgewählt aus: Wasserstoff, Fluor, Chlor, Methoxy, Isopropyl und Cyclopropyl,
bedeuten,
**dadurch gekennzeichnet**, daß man eine Verbindung der allgemeinen Formel (II) 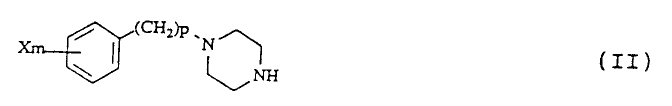 (in der Xₘ und p die oben angegebenen Bedeutungen besitzen) mit einem Halogenreagens der allgemeinen Formel (III) 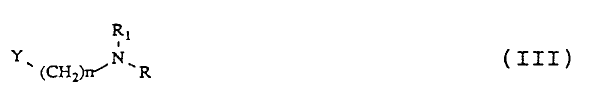 (in der Y ein Halogenatom darstellt, n die oben angegebenen Bedeutungen besitzt und entweder R₁ die oben angegebenen Bedeutungen besitzt und R eine Schutzgruppe für die Aminogruppe, beispielsweise eine Triphenylmethylgruppe, darstellt, oder R₁ und R gemeinsam eine Schutzgruppe, wie die Phthalimidgruppe, bedeuten) in einem aprotischen Lösungsmittel in Gegenwart einer organischen oder anorganischen Base bei einer Temperatur von 40 bis 80°C umsetzt zur Bildung einer Verbindung der allgemeinen Formel (IV) 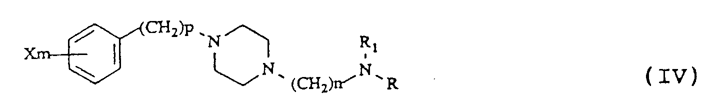 von der man anschließend die Schutzgruppe der endständigen Alkylamingruppe abspaltet: wobei dann, wenn R eine Triphenylmethylgruppe darstellt, man eine Behandlung mit gasförmiger Chlorwasserstoffsäure in einem aliphatischen Alkohol bei einer Temperatur von 0 bis 40°C durchführt; und dann, wenn R und R₁ gemeinsam eine Phthalimidgruppe bilden, man eine Behandlung mit Hydrazin durchführt, so daß man die Verbindung der allgemeinen Formel (V) 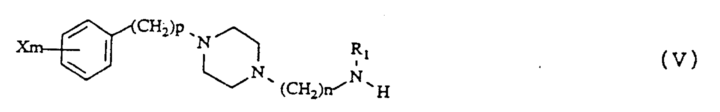 erhält, die man mit der Verbindung der Formel (VI) 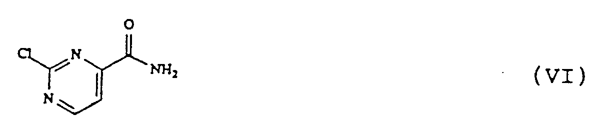 in Gegenwart einer Base bei einer Temperatur von 20 bis 80°C in einem aprotischen Lösungsmittel umsetzt.
